# EUROPEAN PATENT APPLICATION

(11) **EP 4 450 114 A1**
(43) Date of publication of application: **23.10.2024**
(21) Application number: 23168295.6
(22) Date of filing: 17.04.2023
(51) Int. Cl.: A61M 35/00, A45D 24/22, A45D 24/24, A45D 34/04, A45D 40/26, A47L 13/00, B05C 17/00, B43K 5/00, B43K 11/00, A46B 11/00

(54) **INTERNALLY FED APPLIACTOR**

(71) Applicant: GEKA GmbH, 91572 Bechhofen (DE)
(72) Inventor: Schuster, Erwin, Bechhofen (DE)
(74) Representative: Misselhorn, Hein-Martin

(57) **Abstract**

Internally fed applicator (1), with a handle (2) and an applicator unit (6) carried by the latter, to which the agent to be applied with it is fed via the handle (2) or stem into the applicator trim (8) characterised in that the handle (2) or stem has a filling opening (10) which is provided in its circumferential lateral surface and via which feed line (3) can be fed, which feeds the fluid to be applied through the interior of the handle and/or stem to the applicator trim (8) and discharges it in the region of the applicator trim (8) to the applicator unit (6).

## Description

The invention relates to an internally feed applicator according to the preamble of claim 1, an applicator system according to the preamble of claim 13, and the use of an applicator according to the preamble term of claim 14.

### TECHNICAL BACKGROUND

The applicators in question here are used for the application of thin, in most cases water-like pharmaceutical, medical or cosmetic products. Such applicators usually consist of a handle and a bristle section. The bristles of the applicator are used to spread the product over the desired area through stroking and rotating movements. Before application, however, the bristles of the applicator must first be wetted with the product to be applied. There are different ways to apply the product to the bristles.

### STATE OF THE ART

With regard to the way in which the bristles are exposed to the product to be applied, there are essentially two designs for medical or cosmetic applicators.

The bristles can either be wetted with the product from the outside or the product is fed to the bristles through the inside of the applicator or its handle.

In the former case, the bristles can be impacted, for example, by immersing the bristled section of the applicator in a container filled with the product to be applied. Alternatively, it is possible to drip the product to be applied onto the bristles using a pipette or similar. Depending on the application, the product to be applied can also be drizzled directly onto the desired area and the applicator can then only be used for distribution.

Applicators in which the bristles are impacted by the inside of the applicator usually have a cavity inside their stem that extends to the bristles of the applicator. The cavity is or will be filled with the product to be applied and consequently also serves as a reservoir for the product to be applied. In order to convey the product to be applied to the bristles, the applicator is held at an angle and/or slightly compressed in the area of the cavity. Depending on how hard the applicator is squeezed or how long the applicator is held at an angle, more or less product reaches the bristles.

Typically, such applicators are disposable products. The product to be applied is already fed into the cavity during production and after filling the cavity, the handle is closed in such a way that the cavity only has a passage to the bristles.

However, both types of construction are associated with some disadvantages.

On the one hand, there is a relatively high risk of contamination with externally applied applicators. This applies in particular to applicators that are repeatedly immersed in a container with the product to be applied. With these, there is a risk that the entire contents of the container will be contaminated. This can lead to problems, especially with applicators used in the medical or pharmaceutical field.

In addition, exact dosage may be difficult with applicators that are exposed to the product to be applied from the outside. If the applicator bristles are impacted by being immersed in a container with the product to be applied, it is not possible at all or at least difficult to impinge on the bristles with a predefined amount of the product to be applied.

Depending on the application, there is therefore a risk that an excessive amount of product to be applied will accumulate on the bristles. This can lead to wastage of the product or, in the worst case, to misapplication.

However, even with bristles that are drizzled with the product to be applied from the outside, it may be difficult to deliver the correct amount of product to be applied to the bristles. Either the product has to be applied very slowly and carefully, or there is a risk that the bristles are provided with too much product.

Even with internally fed applicators, it is usually difficult to provide the exact amount of product to be applied at the bristles. Especially for inexperienced users, there is a risk that the applicator is held at an angle for too long or - in practice a particular problem - squeezed too tightly, so that an excessive amount of the product to be applied is transported towards the bristles.

In addition, as applicators in which the bristles are exposed to the product to be applied through the inside of the applicator are usually disposable, they are often associated with high waste consumption.

Especially in the case of externally applied applicators or internally applied applicators that cannot be refilled, old residues of the product to be applied can also accumulate in the area of the bristle roots, which are then applied during subsequent application processes. This is unhygienic and can also lead to serious problems in the medical field, for example.

### THE PROBLEM UNDERLYING THE INVENTION

In view of this, it is the problem underlaying the invention to provide an applicator that allows a more precise dosage of the amount of fluid to be applied to the bristles, and preferably as well as a good cleaning possibility of the bristles.

### THE SOLUTION ACCORDING TO THE INVENTION

According to the invention, this problem is solved by means of the features of the main claim directed to the internally fed applicator.

Accordingly, the solution to the problem is provided by an internally fed applicator with a handle and/or at least a stem and an applicator unit carried by the handle or stem.

The agent to be applied with the applicator unit is fed to it via the handle or stem into the applicator trim.

The internally fed applicator is characterised in that the handle or stem has a filling opening through which a feed line can be fed from outside, preferably drop falling down by drop falling down omto the feed opening being preferably designed as a drop collector having a diameter being at least 6 rimes bigger in diameter then a drop provided for it, or by means of a dropper closing the storage container so that preferably no direct fluidal interconnection to the storage container confining the product to be applied is required.

The feed opening is provided in the circumferential surface of the handle or stem.

The supply line leads the fluid to be applied through the inside of the handle and/or of the stem to the applicator trim. In the area of the applicator trim, the supply line delivers the fluid to be applied to the applicator unit.

The applicator can therefore be filled via the filling opening. Theoretically this enables multiple use of the applicator, but the preferred use of the inventive applicator is a one way use. The advantage is that there is a total fluidal separation between the applicator unit and the storing unit, so that it is made sure that the stored fluid stays (best possible) without any contamination.

The point is that such an applicator reduces the risk that the bristles are provided with an excessive amount of fluid to be applied. The amount of fluid to be applied to the bristles can be controlled, preferably twice.

Firstly, already during the filling of the applicator through the filling opening, care can be taken to fill in as little as possible of the amount actually needed. This is because the amount of product fed into the handle can be controlled by the capacity of a funnel or hopper or a basin moulded onto the handle so that it confines the feeding opening.

Even after the applicator has been filled, it is still possible to control the amount of fluid that reaches the bristles.

The inventive applicator can be designed such that the filled fluid is conveyed to the bristles by a corresponding inclination of the applicator Depending on how strongly the applicator is held inclined and/or how long it is held in the corresponding inclined position, more or less fluid can be delivered to the bristles. For this purpose, the "hydraulic resistance" that the supply line in the stem opposes to the flow of the product under the influence of gravity is adjusted accordingly at the factory.

Instead or in addition the applicator can be designed such that the amount of product forwarded by the handle is determined by the capillary action of the handle, it is conceivable to provide a wick for this purpose.

There is also no contamination of the contents of any container filled with the product to be applied.

Another advantage of such an applicator may be that the applicator can be cleaned very easily. The entire area that has come into contact with the fluid to be applied can be rinsed through the filling opening with water or a cleaning agent. In this way, the residues of the fluid to be applied in the area of the roots of the bristles can also be removed.

The applicator is preferably in the form of an applicator for applying eyelash serum. However, it is also conceivable that the applicator is used for applying other products.

In any case, the term "applicator" includes the handle and or stem and the applicator unit. In most cases it includes a handle (in the strict sense), an applicator unit and a stem interconnecting the handle and the applicator unit. It is preferred that the filling opening is embodied in the stem, to make sure that the channel between the filling opening and the bristle covering is kept short.

The term "applicator unit" describes the section of the applicator that both forms the bristles and connects the bristles to the rest of the applicator. The bristles are preferably injection moulded bristles or bristles that are 3-D-printed and that show therefore the according microstructure when examined by means of a microscope.

The applicator unit is ideally a separate element from the handle, which is connected to the handle before use. The connection between the applicator unit and the rest of the applicator can be either permanent or detachable, depending on the application. The latter is particularly advantageous because it makes it easier to clean or replace the applicator unit.

The term "applicator trim" describes preferably the sum of the individual bristles, however (even if not preferred) the "applicator trim" could also be provided by spikes or ribs not forming usual bristles.

An applicator is "internally fed" if it is at least possible to apply the product to be applied to the bristles of the applicator by feeding the product to the bristles at least also through the inside of the applicator.

The terms "product to be applied" and "fluid to be applied" can be used synonymously. Ideally, this is understood to mean a viscous fluid. However, it is also conceivable that the applicator is designed in such a way that it is also suitable for applying low-viscosity fluids that are similar to water in their flow behaviour.

Most preferably the product to be applied is a product with a viscosity (plate/plate) in the range below 200 mPas or even better below 10 mPas.

### ANOTHER UNDERLYING PROBLEM OF THE INVENTION

Another problem underlaying the invention is to provide a system with which the filling of fluid to be applied into the applicator is further facilitated.

### THE FURTHER SOLUTION ACCORDING TO THE INVENTION

The aforementioned problem is solved with an applicator system which consists of at least one applicator according to the invention. In addition, the applicator system consists of a storage container with the fluid to be applied, which is always completely separate from the applicator system. The reservoir is used for dispensing the fluid onto the applicator. The fluid is dispensed onto the applicator through the said filling opening.

The reservoir and the filling opening can be matched to each other in such a way that filling through the filling opening is facilitated and the most accurate dosing possible is made possible.

### ANOTHER UNDERLYING PROBLEM OF THE INVENTION

Yet another problem underlaying the invention is the invention to provide a method by which the amount of fluid to be applied by an applicator can be well dosed.

### THE FURTHER SOLUTION ACCORDING TO THE INVENTION

The aforementioned problem is solved by using an applicator with a capillary according to the invention. The capillary is used to temporarily store a measured dose of fluid to be applied. The intermediate storage serves to deliver the temporarily stored fluid dose gradually into the applicator set over the duration of the intended application time.

This allows the advantages of the applicator according to the invention to be used optimally.

### PREFERENTIAL DESIGN OPTIONS

There are a number of ways in which the invention can be designed to further improve its effectiveness or utility.

It is particularly preferred that the filling opening is bordered by a filling funnel or hopper.

Applicators and their stems are usually relatively thin. Filling the applicator through the correspondingly small filling opening can therefore prove difficult. The funnel, on the other hand, makes filling much easier.

A "funnel" is not necessarily a funnel in the classical sense. Rather, the term merely describes that the filling opening is bordered by an annular surface which is inclined in such a way that a fluid present on the annular surface flows towards the filling opening due to gravity when the applicator is held as intended.

In a further preferred embodiment, the filling device and in particular the filling funnel of the filling device is aligned and designed in such a way that its greatest possible effectiveness is achieved when the applicator is held at an angle. Preferably, the applicator is held with the trim pointing downwards and/or ideally with the longitudinal axis of the handle inclined by 20° to 60° to the horizontal. It is even better if the applicator is held with the longitudinal axis of the handle inclined by 40° to 50° to the horizontal. As long as the applicator is held in the plane or put down, the product to be applied can be filled in via the filling device. Ideally, the product does not flow further in the direction of the bristles, or only to a small extent. As soon as the applicator is brought into a corresponding inclined position, the fluid filled into the applicator continues to flow in the direction of the bristles. This allows the amount of fluid to be applied to the bristles to be controlled twice.

The "greatest possible effectiveness" is given when the fluid flowing in through the filling opening continues to flow in the direction of the applicator unit in the best possible way.

The "filling device" includes the filling opening and the filling funnel.

In a further preferred embodiment, the feed line is a capillary. This delivers the fluid to be applied, which is fed into it via the filling device, into the applicator trim with a more than insignificant time delay.

The capillary effects occurring in the capillary are advantageous because the product to be applied, after flowing into the applicator through the filling opening, does not simply continue to flow uncontrollably in the direction of the bristles and emerge there. Rather, an exit from the capillary in the direction of the bristles takes place as a result of an inclined position of the applicator or only slowly. In the ideal case, the fluid only escapes when the applicator is pressed together in the area of the capillary.

A "capillary" is a thin tube or bore in which capillary effects occur.

Ideally, the filling opening communicates with a storage space in which fluid can be temporarily stored. The storage space can preferably hold a maximum of the portion for a single application process. The fluid temporarily stored in the reservoir is gradually dispensed into the applicator set via the capillary.

The fluid is thus filled in such a way that the fluid first reaches the reservoir through the filling opening either immediately or at least before the fluid reaches the bristles of the applicator. In particular, if the reservoir has a volume that corresponds to the volume of fluid required for an application process, it reduces the risk of more fluid flowing to the bristles than is required for an application process. In any case, the reservoir ensures that the fluid filled into the applicator through the filling opening continues to flow directly towards the bristles. Ideally, the fluid is only released via the capillary into the applicator trim through an inclined position and the gravitation acting on the fluid and/or through slight compression of the applicator in the area of the capillary or the reservoir. All in all, the interaction of the reservoir and the capillary thus provides a twofold possibility for exact dosing. Any misapplication due to the application of too large a quantity of fluid is thus effectively counteracted.

Preferably, the filling opening is so large that the filling level of the storage compartment can be observed through it.

This allows for more precise dosing. The reservoir can first be carefully filled to the desired level. Then the amount of fluid to be delivered to the applicator can be controlled by appropriate handling of the applicator. Ideally, the filling opening also represents the access to the storage space through which the fluid flows into it.

In a further preferred embodiment, the capillary preferably first runs in the applicator handle and then in any case through the coupling section with which the applicator unit is coupled to the handle.

Because the capillary runs through the coupling section, the fluid to be applied can be brought to the bristles in a controlled manner. The capillary can either be designed in such a way that it forms a tube in the area in which it runs through the coupling section of the applicator unit, over which the applicator unit is pushed. However, it is also conceivable that the handle and the applicator unit each have a hole, which together form the capillary when the applicator unit is mounted as intended.

The said "coupling section" of the applicator unit is the area of the applicator unit that is pushed into or onto the handle of the applicator in order to establish a force-fit and/or form-fit connection with the handle.

In another preferred embodiment, the applicator trim is designed to have a combing or separating effect on eyelashes.

For this purpose, the bristles of the applicator are evenly spaced. Thus, when the applicator is used to apply mascara to the eyelashes, the bristles can simultaneously be used to give the eyelashes a desired orientation. In addition, the individual bristles can penetrate into the spaces between the individual eyelashes and thereby also distribute the product to be applied on the facing side surfaces of the eyelashes.

Ideally, the applicator trim consists of preferably injection moulded bristles that are all individually positioned. The individual positioning of the bristles is designed so that each foot of a bristle is spaced apart from each foot of another bristle. The bristles are all individually positioned regardless of how they are made. Optimally, the bristles all have a tapered surface.

As a result of the bristles' tapered surfaces, their tips are relatively thin, while their feet are thicker in relation to the tips. The tips are thus well suited for penetrating into interstices - for example, between the eyelashes. The thicker feet, on the other hand, ensure that the bristles come into contact with the side surfaces of the interstices into which the individual bristles have penetrated and distribute the fluid to be applied evenly on these side surfaces. In addition, the thicker feet ensure that the bristles are not too bendy.

The fact that the bristles are "individually positioned", i.e. their feet are spaced apart from each other, does not exclude that each foot of a bristle merges integrally into the applicator unit.

The terms "foot" of a bristle and "root" of a bristle are synonymous.

In a further preferred embodiment, the applicator has a flattening and/or widening at its end facing away from the applicator trim. The flattening and/or widening is designed in such a way that the applicator, held between two fingers, can be brought to the surface to be applied without fear of unintentional rotation of the applicator.

This enables safe handling and precise guidance of the bristles along the desired area.

A "flattening" is understood to be a section that has a smaller thickness than the section of the applicator adjacent to it and also has a non-circular cross-section.

A "widening" is a section that has a greater thickness than the section of the applicator adjacent to it and also has a non-circular cross-section.

The non-circular "cross-section" of the widening or flattening is to be understood as a cut surface which runs parallel to the cross-section of the applicator handle, so that the longitudinal axis of the applicator handle is in any case partially aligned orthogonally to the cut surface of the non-circular cross-section.

In the case of a widening at the end of the applicator, this can, for example, be designed as a torus whose outer diameter is larger than the diameter of the part of the applicator adjacent to the torus. In this case, the hole in the centre of the torus additionally contributes to safer handling of the applicator, as a finger can be fully or partially inserted into the hole to prevent unwanted rotation of the applicator.

In another preferred embodiment, the filling funnel has a bowl section shaped like a steep curve on the side of the distal end of the stem facing away from the applicator unit and/or a pointed drop guide surface on the side of the proximal end of the stem facing toward the applicator unit.

The said bowl section ensures that all drops that touch down to the stem above the filing opening run down into the filling opening more quickly.

The said drop guide surface facilitates the dripping of fluid and preferably runs in the shape of a ship's bow, like a ship's bow pulled far upwards. It avoids or makes it more difficult that drops run down the outer surface of the of the stem in order to meet the bristle covering from outside.

If, during filling, some of the fluid is accidentally poured not exactly into the filling opening, but next to it, the drop guide surface ensures that the fluid poured next to it nevertheless flows in the direction of the filling opening and finally into the inside of the applicator. This applies at least as long as the applicator is held as intended.

The drop guiding surface is "ship bow-shaped" if the projection of the edge delimiting the drop guiding surface into the plane passing through the central longitudinal axis of the applicator and dividing the feed hopper into two symmetrical halves connects a highest point and a lowest point with by a concave line. The concavity here refers to the rest of the feed hopper.

Ideally, the feed funnel is closer to the distal end of the stem than to the proximal end.

The fluid to be applied must therefore flow through a shorter section to reach the bristle shoulder. This reduces the time needed for an application process.

Here, the "distal" end of the stem represents the end of the stem facing the applicator trim, while the "proximal" end of the stem represents the other end of the stem.

### FIGURE LIST

Fig. 1 shows a perspective view of an applicator according to the invention.
Fig. 2 shows the applicator in sectional view
Fig. 3 shows the applicator in plan view
Fig. 4 shows the applicator in side view
Fig. 5 shows the applicator in front view
Fig. 6 shows a storage container of an applicator system according to the invention.
Fig. 7 shows a another preferred embodiment differing from the first one only in regard to its almond-like bristle carrier.

### PREFERRED EMBODIMENT

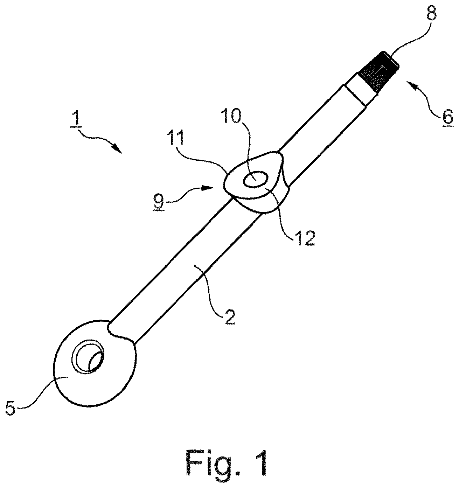

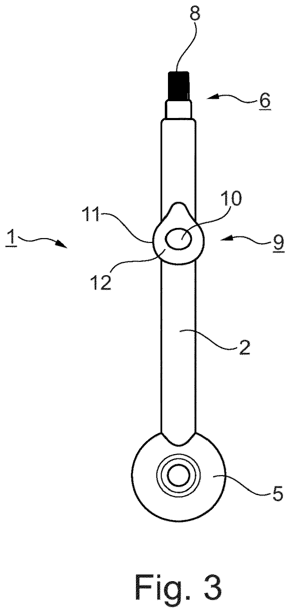

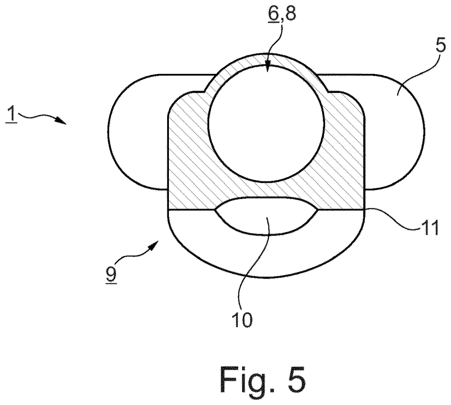

The mode of operation of the invention is explained by way of example with reference to Figs. 1-6.

The applicator 1 is held on the handle 2. The torus-shaped widening 5 at the proximal end of the handle 2 serves to ensure safe handling of the applicator 1.

The individual bristles of the applicator attachment 8 are used to apply a fluid to the desired area.

In order to start intended use the fluid to be applied is filled into the applicator 1 or the applicator handle 2 via the filling device 9.

The filling device 9 comprises a filling opening 10 in the outer circumferential surface of the handle 2, as well as a filling funnel 11.

The filling funnel 11 edges the filling opening 10 and is equipped with a drop guide surface 12. Starting from the outermost edge of the filling funnel 11, the drop guiding surface 12 initially extends in an annular manner around the filling opening 10. The individual incremental sections of the annular area of the drop guide surface 12 each have a (negative) gradient inclined towards the filling opening.

The slope of the drop guide surface 12 is greatest in the bowl shaped area of the filling funnel 11 facing away from the applicator attachment 8 and decreases towards the area of the filling funnel 11 facing the applicator attachment 8. In the section immediately adjacent to the filling opening 10, the drop guide surface 12 can merge vertically into the filling opening 10. In this area, the drop guide surface 12 or the outer edge of the filling funnel 11 surrounding the drop guide surface 12 converges to a point.

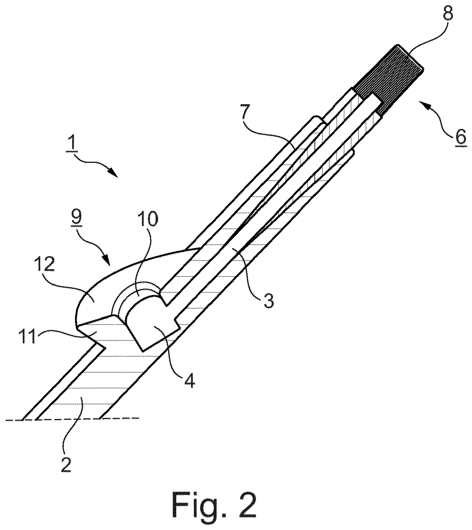

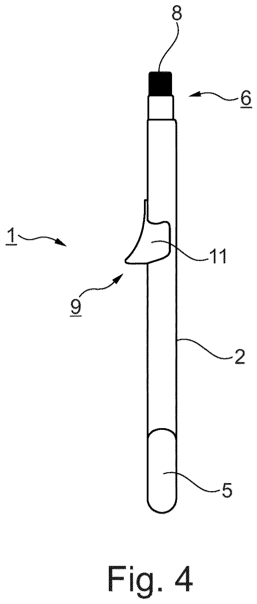

As can be clearly seen from the sectional view shown in Fig. 2, the filling funnel 11 merges integrally into the stem 2. On the side facing away from the applicator trim 8, the filling funnel 11 has a greater height in relation to the filling opening 10 than on the side facing the applicator trim 8. Starting from the maximum height in the area of the filling funnel 11, which is diametrically opposite the applicator attachment 8, the height of the filling funnel 11 decreases continuously towards the end facing the applicator attachment 8. The decrease in height is not linear, but such that the outer edge of the filling funnel 11, which delimits the drop guide surface 12, has a concave curvature in its course. This can be seen clearly in the side view shown in Fig. 4.

The filling funnel 11 facilitates the filling of the fluid to be applied into the filling opening 10. Thus, even the part of the fluid that is not poured exactly into the filling opening but onto the drop guide surface is ultimately directed towards the filling opening 10 and reaches the inside of the applicator 1.

After the fluid has passed through the filling opening 10 into the interior of the applicator handle 2, it accumulates in the reservoir 4 before it is conducted further through the supply line 3, designed as a capillary 3, in the direction of the applicator unit 6. The supply chamber 4 extends further into the applicator shaft 2 than to its longitudinal axis. The supply line 3, on the other hand, runs approximately coaxially to the longitudinal axis of the applicator shaft 2. This ensures that the fluid flowing into the supply chamber 4 cannot immediately continue to flow through the supply line 3, but first accumulates at the bottom of the supply chamber 4. This offers the possibility to fill only a small amount of fluid to be applied into the supply chamber 4, which only flows further in the direction of the applicator unit 6 when the applicator 1 is tilted. This ensures that not too much of the fluid to be applied reaches the bristles of the applicator 1.

The supply line 3 connects the supply chamber 4 with the applicator unit 6. The applicator unit 6 forms on the one hand the applicator trim 8 and on the other hand the coupling section 7. The coupling section 8 is the area of the applicator unit 6 that is inserted into the stem 2 of the applicator. In its centre, the coupling section 7 of the applicator unit has a through hole. This connects to the supply line 3 and directs the fluid coming from there further in the direction of the applicator trim 8. The applicator trim 8 is formed by several individual bristles which merge integrally into the remaining part of the applicator unit 6. With the help of the applicator attachment 8, the fluid arriving there is finally applied to the desired location.

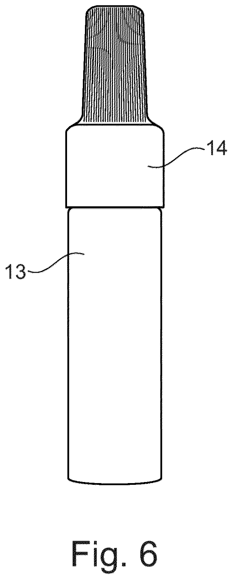

Fig. 6 also shows a reservoir 13 for the fluid to be applied, closed by a lid 14. The reservoir 13 and the applicator 1 together form an applicator system.

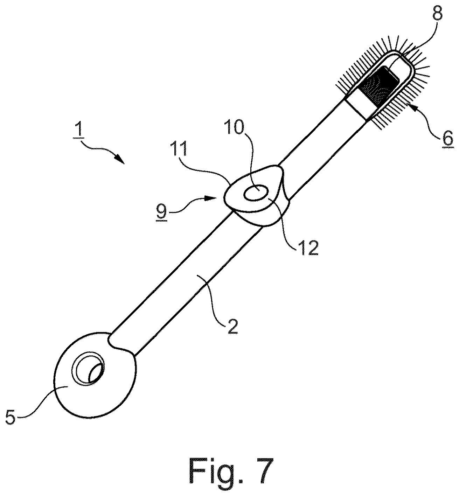

Fig. 7 shows a another preferred embodiment differing from the first one only in regard to its almond-like bristle carrier which carries a bristle trim on its circumferential surface instead on its front face only.

### MISCELLANEOUS

Protection is sought, too, for an internally fed applicator, with a handle and an applicator unit carried by the latter, to which the agent to be applied with it is fed via the handle into the applicator trim, being special in that the handle has a filling opening which is provided in the in the surface of its distal (away from bristle trim) end face and via which a feed line can be fed, which feeds the fluid to be applied through the interior of the handle to the applicator trim and discharges it in the region of the applicator trim to the applicator unit.

### LIST OF REFERNCE NUMBERS

- 1: Applicator
- 2: Handle
- 3: Feed line / capillary
- 4: Storage room
- 5: Widening (of the applicator handle)
- 6: Applicator unit
- 7: Coupling section
- 8: Applicator trim or, more specific, bristle trim
- 9: Filling device
- 10: Filling opening
- 11: Filling funnel or hopper
- 12: Droplet guide surface
- 13: Storage container
- 14: Lid of the storage container

## Claims

1. Internally fed applicator (1), with a handle (2) and an applicator unit (6) carried by the latter, to which the agent to be applied with it is fed via the handle (2) or stem into the applicator trim (8) **characterised in that** the handle (2) or stem has a filling opening (10) which is provided in its circumferential lateral surface and via which feed line (3) can be fed, which feeds the fluid to be applied through the interior of the handle and/or stem to the applicator trim (8) and discharges it in the region of the applicator trim (8) to the applicator unit (6).

2. Internally fed applicator (1) according to claim 1, **characterised in that** the filling opening (10) is bordered by a filling funnel (11).

3. Internally fed applicator (1) according to claim 1 or 2, **characterised in that** the filling device (9) and in particular the filling funnel (11) is aligned and designed in such a way that its maximum effectiveness is achieved when the applicator (1) is held at an angle, preferably with the trim (8) facing downwards and/or ideally with the longitudinal axis of the handle inclined by 20° to 60°, preferably by 40° to 50°, to the horizontal.

4. Internally fed applicator (1) according to one of the preceding claims, **characterised in that** the feed line (3) is a capillary (3) which discharges the fluid to be applied, fed into it via the filling device (9), into the applicator trim (8) with a time delay.

5. Internally fed applicator (1) according to claim 4, **characterised in that** the filling opening (10) communicates with a reservoir (4) which preferably accommodates at most the portion for a single application operation and in which fluid can be temporarily stored which is gradually dispensed into the applicator trim (8) via the capillary (3) .

6. Internally fed applicator (1) according to one of the preceding claims, **characterised in that** the filling opening (10) is so large that the filling level of the reservoir (4) can be observed through it.

7. Internally fed applicator (1) according to claim 5, **characterised in that** the capillary (3) preferably first runs in the applicator stem (2) and then in any case through the coupling section (7) with which the applicator unit (6) is coupled to the stem (2).

8. Internally fed applicator (1) according to one of the preceding claims, **characterised in that** the applicator trim (8) is designed in such a way that it can develop a combing or separating effect towards eyelashes.

9. Internally fed applicator (1) according to one of the preceding claims, **characterised in that** the applicator trim (8) consists of preferably injection moulded bristles which, also independently of their method of manufacture, are all individually positioned so that each foot of a bristle is spaced from each foot of another bristle, the bristles optimally all having a tapered lateral surface.

10. Internally fed applicator (1) according to one of the preceding claims, **characterised in that** the applicator (1) has, at its end facing away from the applicator trim (8), a flattening and/or widening (5) which is designed in such a way that the applicator (1), held between two fingers, can be brought up to the surface to be applied without there being any fear of unintentional rotation of the applicator (1) .

11. Internally fed applicator (1) according to one of the preceding claims, **characterised in that** the filling funnel (11) has, on the side of the distal stem end facing away from the applicator unit (6), a tapering drop guide surface (11), preferably running in the shape of a ship's bow, which facilitates the dripping in of fluid.

12. Internally fed applicator (1) according to any one of the preceding claims, **characterised in that** the feed hopper (11) is closer to the distal end of the stem (2) than to the proximal end.

13. Applicator system comprising at least one applicator (1) according to one of the preceding claims and a reservoir (13), always completely separate therefrom, containing the fluid to be applied for dispensing the fluid onto the applicator (1) through said filling opening (10).

14. Use of an applicator (1) with a capillary (3) according to one of the preceding claims for the intermediate storage of a fluid dose to be applied in a measured manner, in order to deliver it gradually into the applicator trim (8) over the duration of the intended application time.
